# EUROPEAN PATENT SPECIFICATION

(11) **EP 4 157 493 B1**
(45) Date of publication and mention of the grant of the patent: **05.11.2025**
(21) Application number: 21813359.3
(22) Date of filing: 25.05.2021
(51) Int. Cl.: B01D 53/62, B01D 53/77, B01D 53/78, B01D 53/79, B01D 53/80, C01F 11/18, C01B 32/60, C12M 1/06, C12M 1/00, C12M 1/02

(54) **PHOTOLYTIC BIOREACTOR SYSTEM AND METHOD**
PHOTOLYTISCHES BIOREAKTORSYSTEM UND VERFAHREN
SYSTÈME ET PROCÉDÉ DE BIORÉACTEUR PHOTOLYTIQUE

(30) Priority: 27.05.2020 US 202063030916 P
(43) Date of publication of application: 05.04.2023
(73) Proprietor: Sammy, Johann, Q., Weymouth, MA 02191 (US)
(72) Inventor: Sammy, Johann, Q., Weymouth, MA 02191 (US)
(74) Representative: Zanettin, Gianluigi
(86) International application number: PCT/US2021/034046
(87) International publication number: WO 2021/242748

(56) References cited:
- CN-A- 102 923 749
- CN-B- 105 502 462
- CN-B- 105 502 462
- US-A1- 2010 068 109
- US-A1- 2010 068 109
- US-A1- 2014 209 544
- US-A1- 2014 209 544
- US-A1- 2014 341 792
- US-A1- 2014 341 792
- US-A1- 2017 218 407
- US-A1- 2017 218 407

## Description

### FIELD OF THE DISCLOSURE

This disclosure relates to the utilization of CO₂, and other gases, with mineral feedstock to synthesize products.

### BACKGROUND OF THE DISCLOSURE

The excess generation of CO₂ in human activity has resulted in a number of problems of human and animal life. The continued increase in emission of CO₂ from industrial activity gives rise to a series of risks and detrimental effects. These include the impact on the radiation balance of the atmosphere, the so-called "greenhouse" effect or climate change as an exemplary consequence of increasing CO₂ emission. This carbon overload creates the greatest risk of irreversible changes if it continues to accumulate unabated in the atmosphere. Much technological development is centered on reducing the emission of CO₂. However there is also a technological movement of capturing and using CO₂ for beneficial purposes. One such technical area is in methods for utilizing CO₂ in bioreaction with mineral feedstocks to synthesize products. The synthesizing of products can be enhanced by introducing into the bioreactive process the addition of photolysis in combination with general mixing of CO₂ with the selected feedstock.

CN105502462B discloses a device and a method to prepare nano-calcium-carbonate using a photolytic bioreactor including a microporous pipeline, an air inlet pipe and suspended calcium hydroxide liquid flow in said venturi reaction tube. The venturi tube being sequentially communicated with an entrance, a contraction section, a venturi diffuser, a liquid outlet of the fluid.

### BRIEF DESCRIPITION OF THE DRAWINGS

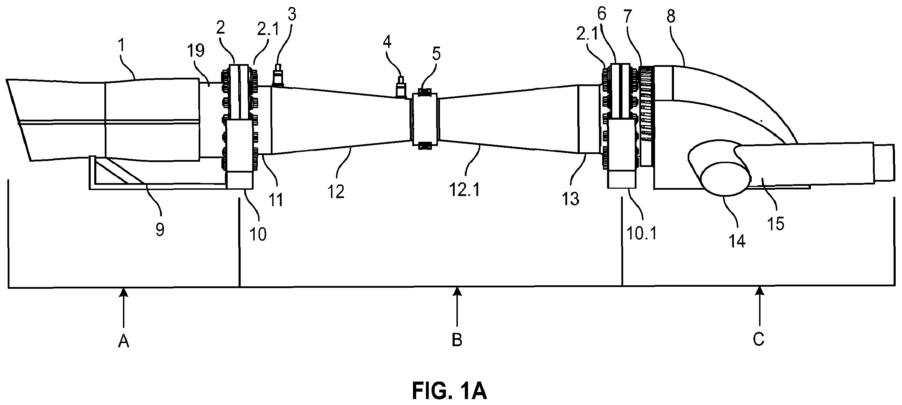

FIG. 1A illustrates the primary components of the disclosed reactor which include the venturi nozzle, flanges, electric motor housing, UV-B/C exhaust fairing and shield, and exhaust nozzle. In addition, the general arrangement of the pressure taps, and support stands are presented in a 2D right side elevation view.

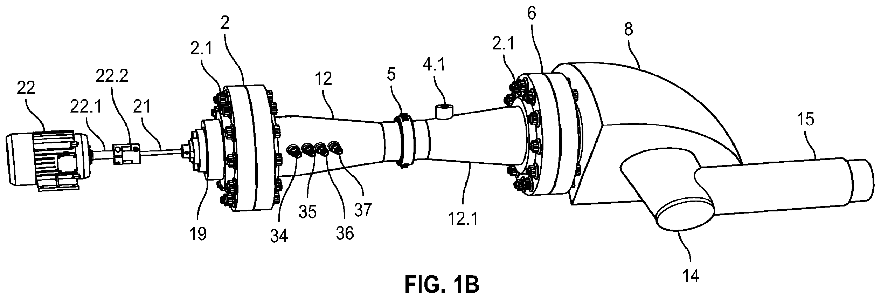

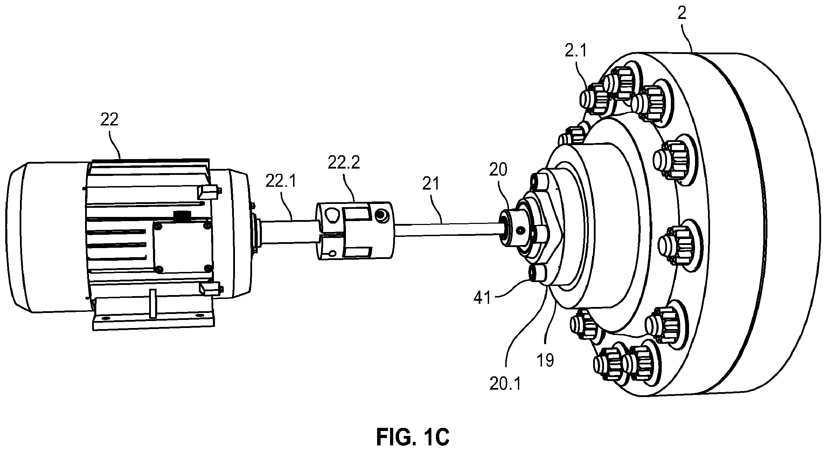

FIG. 1B is an isometric right-side view of the reactor assembly of Figure 1 with 3D detail of the electric motor, shaft coupling, main drive shaft, and flanged cap, support assembly, and rear distant view of the UV exhaust fairing, shield, and exhaust nozzle.
FIG. 1C is a closeup isometric view of the electric motor, without the motor fairing - housing, and depicting the shaft coupling, main drive shaft, seal-bearing assembly, and cap end flange section in 3D detail.

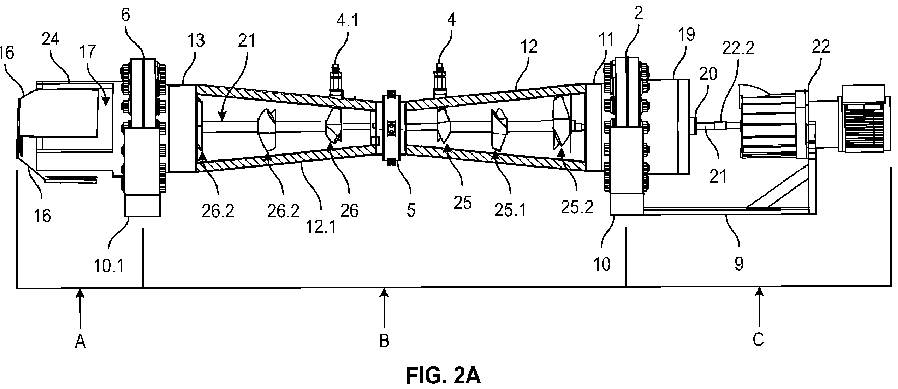

FIG. 2A is a cross-section, left side elevation view of the reactor venturi cavity of FIG. 1. Shown in the 2D cross-section view are propellers for mixing and agitating the slurry mix and the main drive shaft extension throughout the venturi nozzle. Displayed in a side elevation view showing a crucible formed of walls from quartz, sapphire, or other type of hardened clear or translucent material that has high UV transmissivity and is enclosed by the ultra-violet lighting array mounts, and cap end flange retainer. Further depicted are the electric motor, electric motor - reactor mounts and cap end flange assembly, and ports in the side elevation view.

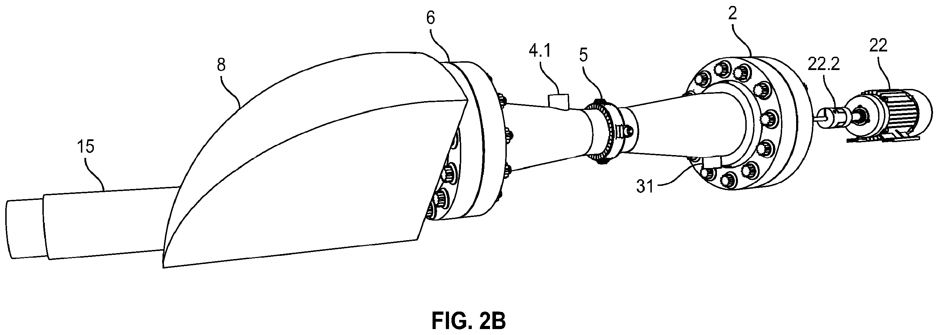

FIG. 2B is a left side perspective view of the complete bioreactor assembly, without the electric motor fairing-cover of FIG. 1A.

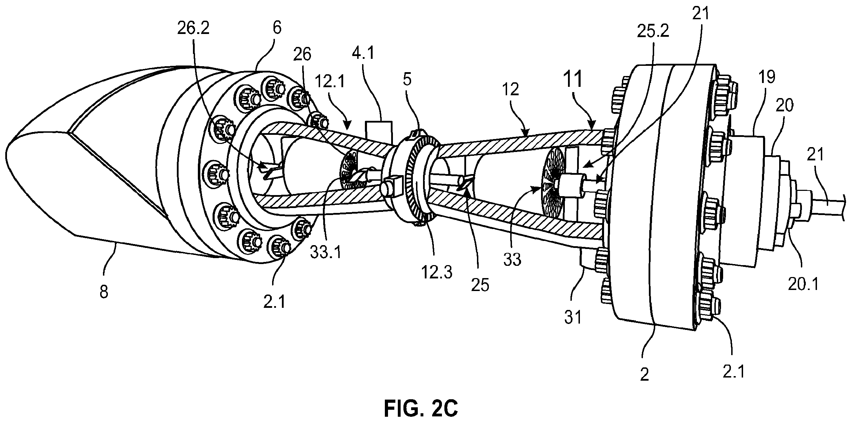

FIG. 2C is an oblique drawing of the left side of the bioreactor beginning at the main drive shaft and ending at the UV exhaust nacelle and shield. Also pictured is a sectional view of the venturi nozzle cavity, illustrating perforated containers mounted to the inner walls and oriented about the main drift shaft. The section view also features the mixer-agitators mounted to the main drive shaft and housed within the reactor cavity.

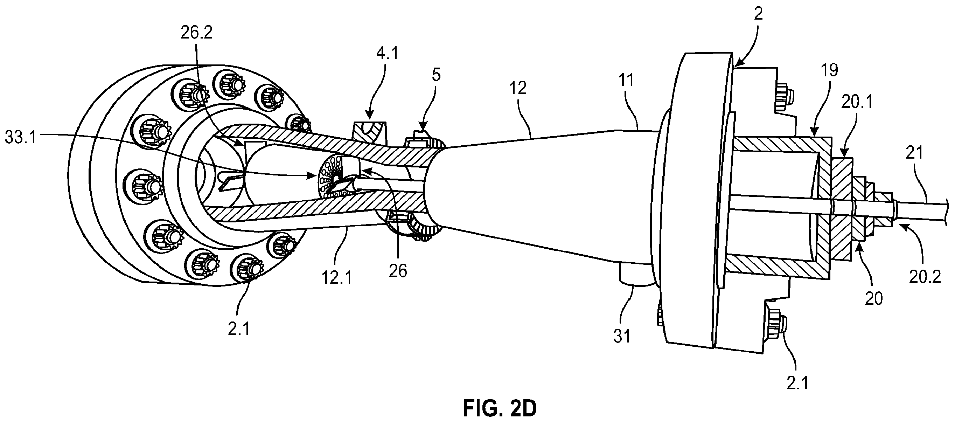

FIG. 2D is an oblique drawing of the left side of the bioreactor beginning at the main drive shaft and ending at the split wall retaining cap end flange. Depicted in a sectional view of the venturi nozzle cavity are perforated containers mounted to the inner walls of the bioreactor and oriented along the main drift shaft. The section view also illustrates, the mixer-agitators, mounted to the main drive shaft and housed within the reactor cavity, port, heating band-element, flanged cap ends, straight section tube, and seal-bearing assembly configuration.

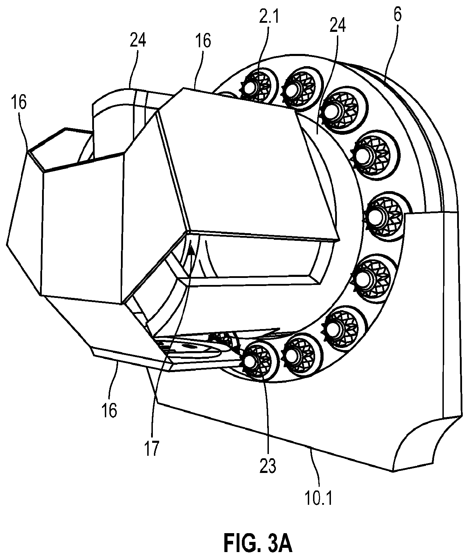

FIG. 3A is perspective left side view of the UV and visible light LED's, light emitting diodes and lamps array installed around the split retaining wall cap end supported by block mounts and flanges. The UV exhaust fairing and shield is removed and not shown. Also depicted is the quartz or sapphire cylinder or other type of clear material cylinder housed within the split wall cap end.

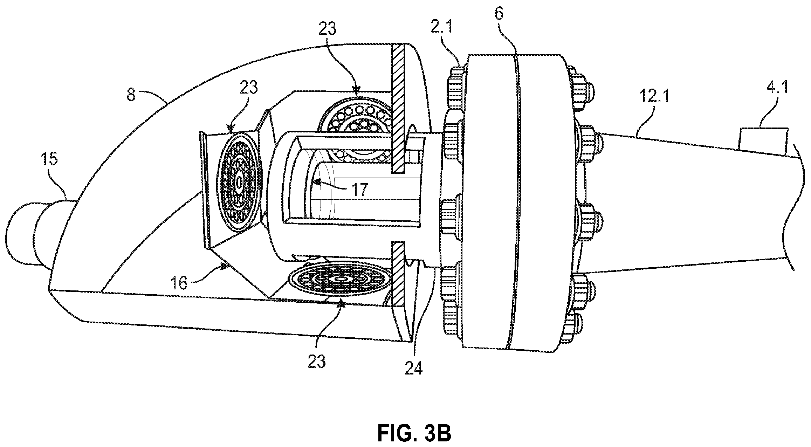

FIG. 3B is a dimetric left side view of the split retaining wall cap end flange, left section of the venturi nozzle bioreactor and port. The sectional view of the UV exhaust-shield nacelle reveals the placement and orientation of the UV emitter housing, UV emitters, quartz, sapphire, or clear material crucible housed within the split retaining wall cap end flanges.

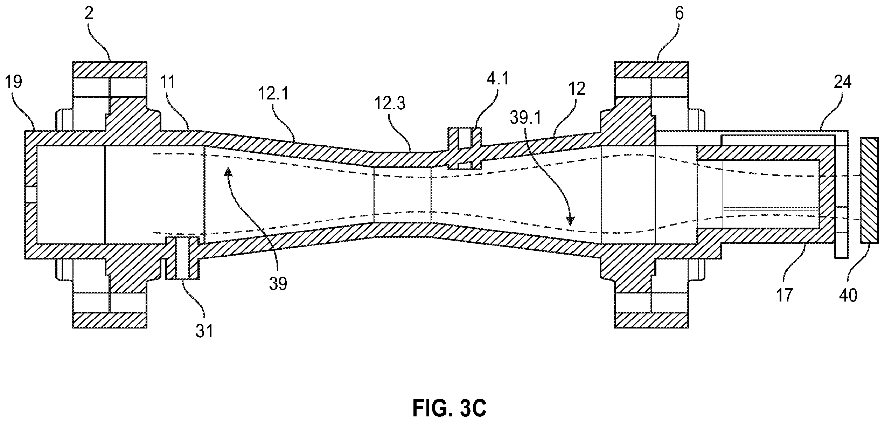

FIG. 3C is a 2D right side elevation, and cross-section view of the reactor body and components. The cross-section view begins (left to right) at the flanged cap end and ends at a UV emitter source for fiber optic cables. In the cross section view of the reactor cavity, without electric motor and UV fairing covers, fiber optic cables are illustrated, beginning at the UV emitter mounted outside the split wall retaining flanged cap end, and passing through the reactor cavity.

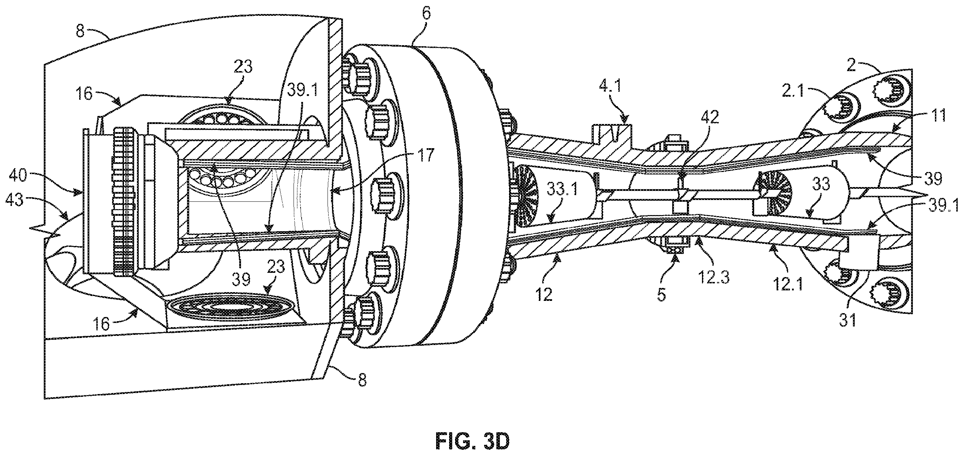

FIG. 3D is a left side perspective view of the bioreactor that depicts, cross-sectional views of the UV and visible light nacelle-exhaust shield, and cross-section view of the venturi nozzle reactor body core or cavity. Inside the core or cavity of the reactor are fiber optic cables that begin at the UV or visible light emitter source and are mounted to the interior walls of the bioreactor. Inside the UV nacelle the LED, or lamp-based UV and visible light source are shown. The fiber optics begin at the UV or visible light source emitter, and pass through the cap end flange and crucible, entering the reactor cavity.

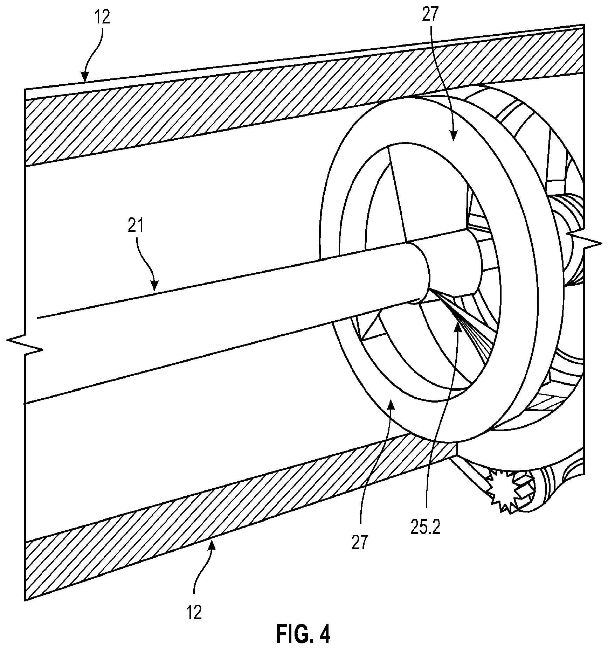

FIG. 4 is a detailed, perspective view of the propeller blade tip ring rotor and stator housing with windings that form a permanent magnet generator. In this configuration, the propeller blades rotation generates electrical energy which can be stored for future power consumption or converted to usable electricity to operate the electric motor. This configuration facilitates energy recovery during the reaction time or post-reaction time.

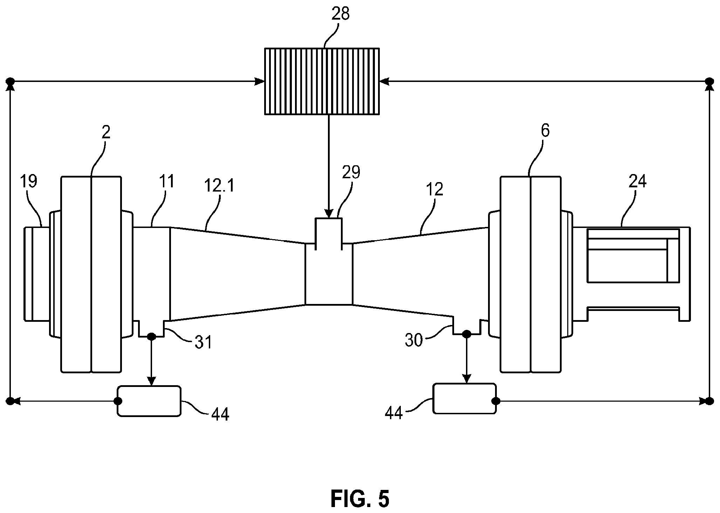

FIG. 5 is a 2D right side elevation view of the primary reactor assembly with multiple pressure tap ports to recover pressure energy. A liquid-gas boost pump assembly is depicted, which increases the pressure of the waste CO₂ and water vapor or steam that is re-injected into the reactor to induce rotation of the propeller mixer, purge the product of the reaction or the carbonates from the reactor, and to recycle CO₂ that may have not carbonated with the aqueous slurry feedstock.

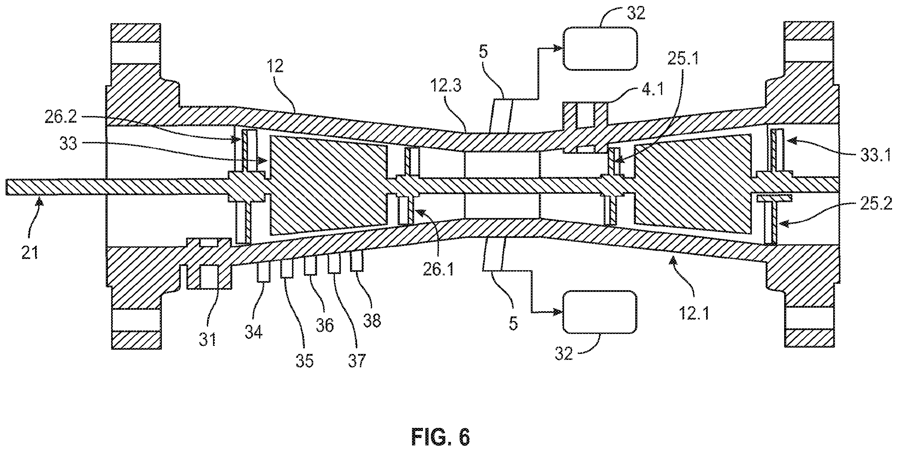

FIG. 6 is a 2D right side elevation view of the reactor. Cross section views of the venturi nozzle reactor depict perforated cylinders for the storage of organic and inorganic media housed within the cavity of the reactor, oriented around the main drive shaft. Additionally, a multi-port injection system for contactless feedstock loading is depicted. The perforated containers house either mineral feedstock or cell tissue cultures and the perforations allow for diffusion via circulation of the mixer turbines, of the organic nutrient media and inorganic feedstocks injected into the reactor cavity, through multi-ports.

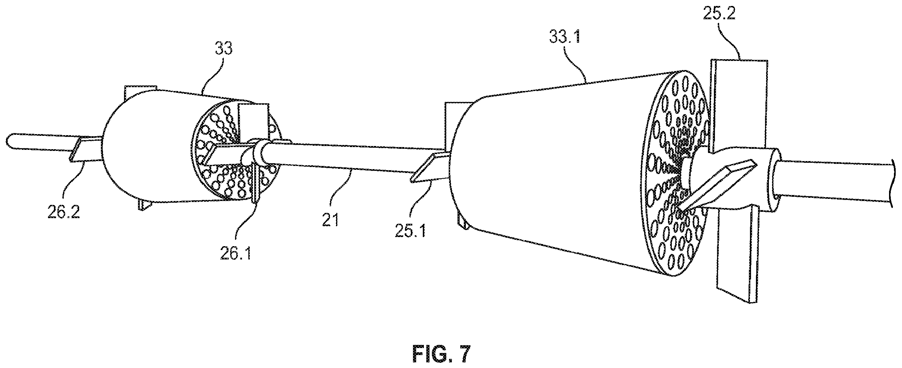

FIG. 7 is a dimetric, close view of the perforated cylinders for housing organic and inorganic media. The cylinders are oriented along the main drive shaft and receive liquids, solids and gaseous media flow circulated from the cavity of the reactor by mixer turbines.

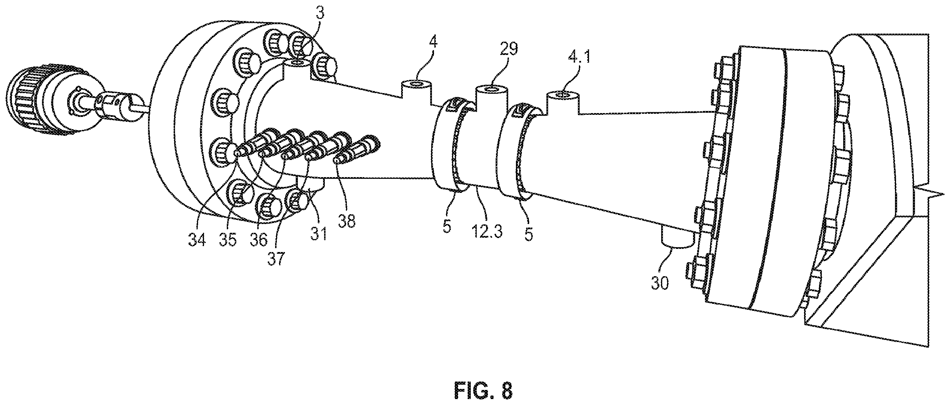

FIG. 8 is a dimetric view and spatial location reference of injection and exhaust ports of arbitrary configuration mounted along the outer walls of the Venturi Nozzle Section of the bioreactor. The ports are used for the injection and ejection of liquids, solids and gases into and out of the bioreactor.

In the above figures section illustration is shown by section fill lines.

### DESCRIPTION

This disclosure presents methods for utilizing CO₂, and other gases, with mineral feedstock to synthesize products. The synthesized products, as the result of liquid, solid, gas photo-chemical reactions within the advanced bioreactor of the disclosed embodiment, are precipitated raw material for multiple end use consumer and industrial products. Waste heat, pressure and torque produced from the bioreactor is utilized for generating electricity and or heat exchange for environmental conditioning through a combination of energy recovery devices for energy efficiency. Energy recovery devices offsets and lower the cost of operating the reactor as the disclosed reactor integrates photolysis via ultra-violet light, as an integral component, of a reactor system, composed also of an active mixer-agitator assembly, pressure and vacuum vessel chamber, heat source, and ports for media ingestion. The disclosed reactor is designed to simulate a wide range of environmental conditions that are conducive to transforming gaseous, solid, and liquid feedstock, like carbon dioxide - CO₂, and other feedstocks that are inorganic and/or organic in an aqueous medium, into inorganic and organic product.

Carbonates, CO₃, are the result of photo and chemical dissociation of compounds and subsequent binding into compound carbonate form through ion exchange. The result of such photo-chemical, electro-chemical and natural kinetic reactions binds CO₂ carbon dioxide with inorganic mineral silicates, in long term, stable form as carbonates. Sources of CO₂ may come from, but are not limited to, direct air capture of CO₂ from the free stream atmosphere, and or capture from exhaust, flue gas of industrial plants, factories, and buildings. More particularly, the present disclosure describes integration of an energy recovery device as part of the disclosed reactor. Potential products resulting from photo-chemical reactions within the advanced bioreactor of the present embodiment, may include but are not limited to, inorganic carbonates, fluid and gel medium, gases, cell and plant cultured foodstuff, reagents, solid inorganic oxides, mineral feedstocks, and organic bodies like enzymes and microbes, for human and non-human utility and consumption.

Generally, the products derived from photo-chemical reaction in the advanced bioreactor of the disclosed embodiment, can be broadly categorized as liquids, solids and gaseous product for human and non-human consumption and utility.

The disclosed bio reactor with photolytic and energy recovery capability is used, but is not limited to, carbonation of CO₂ with minerals and additives under high temperature and high CO₂ partial pressure, which is also denoted as P _{(CO2)}. CO₂ is a greenhouse gas and the primary gas released into the atmosphere responsible for the climate crisis. The IPCC- International Panel on Climate Change has set targets and has called for the active removal of CO₂ through direct air capture from a myriad of technical and natural solutions, many of which do not yet exist. The reactor disclosed utilizes direct air captured CO₂, industrial emitter CO₂, or separated CO₂ from an industrial flue gas waste stream and transforms CO₂ through photo and chemical reactions into carbonates, and in the process, provides a stable long term storage solution and use for sequestered CO₂. This type of carbonation is known as accelerated weathering or enhanced weathering. A similar process occurs naturally when either gaseous CO₂ or carbonated water, which is water enriched with CO₂, reacts with silicates and other minerals to produce partial carbonates by volume and weight. However, the time scales for natural carbonation are hundreds and thousands of years, if not several thousand millennium or more.

The disclosed photolytic reactor with energy recovery can complete a full carbonation batch of CO₂ within minutes and hour(s) and offset its energy consumption with energy recovery devices. Other types of inert, noble, allotrope, and chalcogen group gases, that can be utilized as feedstock within the reactor include, Nitrogen (N₂), Argon(Ar), Krypton (Kr), Ozone (O₃) and Oxygen (O₂).

Generally, bioreactor types fall into 2 broad flow categories: horizontal and vertical. Processing flow, either vertical or horizontal comes with advantage and drawbacks. Vertical reactors are structural designs of a cylinder housing, usually steel alloy material, and include a mixer/propeller (single stage) with sufficient space to house media, like minerals, additives, and CO₂, including head space. The disclosed photolytic reactor with energy recovery devices, can be pressurized by a gas booster pump that injects CO₂ up 2200 pounds per square inch, referred to by abbreviation, psi, and expressed as P _{(CO2)} throughout the description of the disclosed embodiment. The advantages of vertical reactors include simple construction that facilitates scaling to sizes of 150 cubic meter volume and higher.

Vertical reactors can house mixers or impellers that agitate the slurry and or media under reaction There are several disadvantages to vertical type reactors. Firstly, gravity is always pulling the slurry downwards causing un-swirled or un-agitated media to sink to the bottom where carbonation may occur slowly or not at all. Secondly, a single mixer would struggle to actively mix media that has sunk to the bottom economically. Without persistent agitation, a passivating silicone layer will form that inhibits carbonation of silicates slowing reaction kinetics, namely the absorption and subsequent carbonation of CO₂. Thirdly, as volume and mass of the media and/or slurry per batch increase, slurry and/or mediaweight may make vertical mixing a slower process than expected at 150 cubic meter volume scale and larger, which may result in efficiency losses in per batch throughput.

Horizontal Axis, 1-Step Reactors hold key advantages over vertical, stirred reactors. The first advantage is the efficient use of capacity by volume. The entire volume of the reactor is available for carbonation with distributed weight displacement of the slurry, increase in slurry particle surface area from horizontal spreading and mixing, unlike a vertical type of reactor. The second advantage is improved flow dynamics (pressure) from a horizontal venturi nozzle, venturi type design as compared to a vertical, cylindrical and/or spherical geometry- type design.

The present reactor in a central pressure body portion employs a venturimeter structure having a throat or converging part that lies between converging and diverging parts of the venturi. The throat portion defines the narrowest part through which fluid flow converges and flows at increased velocity and decreased pressure. The diverging part extends away from the throat, increasing in cross section wherein the fluid flowing through it diverges.

Horizontal flow reactors perform at a higher throughput per batch since the geometry of the nozzle results in efficient flow per volume of the slurry and/or media through the constricted section of the convergent-divergent venturi nozzle design. The basic physics are defined by the Bernoulli effect and the Venturi effect. As the mixers transfers the aqueous slurry to the right and left, side of the venturi reactor, and right to left from reversing electric motor rotation, the slurry or media must pass through a constricted section of the reactor which results in an increase in velocity and drop in pressure of the slurry in transit. The heat band or heat element is placed around the outside surface of the constricted section which then heats the core of the reactor, and in turn heats the slurry in transit through the constricted section of the venture nozzle reactor.

Active mixing passes the aqueous slurry through the heated, constricted section of the venturi. Heat is then distributed, evenly and faster throughout the reactor, relative to a cylindrical, spherical, or non-venturi reactor body design. The increase in heating distribution results in faster times to optimal reaction temperature.

Moreover, the present embodiment incorporates a plurality of ultra-violet (UV)-A, UV-B and UV-C light emitting diodes, LED, excimer lamps, lamps, and to speed up dissociation of the minerals, gas, and additives in the slurry, within the cavity of the reactor vessel. As light energy approaches shorter wave lengths, it becomes more energetic. This light energy and intensity in the far, short, and vacuum ultra-violet, VUV, range can dissociate chemical compounds, in the presence of other gases, and independently based upon local reactor heat, and pressure conditions. UV-C and UV-B light energy provide an ancillary benefit as germicidal agents during the reaction process. UV-C light is particularly noted for its ability to neutralize germs, bacteria, viruses, and other pathogens. The complete ultraviolet wavelength of the UV and visible light emitters, diodes, and lamps integrated into the bioreactor, range from vacuum ultraviolet (VUV) of 100nm to 200nm, UV-C from 200nm-280nm, UV-B from 280nm-315nm, and UV-A from 291nm- 400nm, as well as visible light from 400nm-700nm. All disclosed UV and visible light emitters are non-ionizing.

UV-C is a highly energetic spectrum of ultraviolet light that is non-ionizing, electromagnetic radiation at wavelengths between 125-200 nanometers (nm). UV light is used in three major capacities in the advanced bioreactor of the disclosed embodiment which include, (a) water purification for carbonation enhancement from OH (hydroxyls) and O₃ (ozone) produced by UV-C; (b) generation of fast reacting OH (hydroxyls) that bind with the leached minerals, calcium or magnesium, or other inorganic and organic feedstock, to create hydroxides, and (c) UV-C generated OH can be reacted with CO (carbon monoxide) and O (Oxygen) which are by-products of UV-C at a wavelength of 160nm, then recombined to create CO₂ products for use in mineral carbonation within the reactor or for storage and use elsewhere.

General equations governing photo-chemical reaction kinetics under specific heat, pressure, resident time, ultraviolet dosing, solid mineral feedstock, gas inputs, and general products of the bioreactor of the disclosed embodiment are shown in the following paragraph. All inputs in the described reaction are inorganic and do not consider organic inputs for bio-mineralization or bio-carbonation for enhanced weathering of inorganic silicates.

The mineral wollastonite is used as in example 1. Wollastonite, with a general chemical compound of calcium silicate, CaSiO₃, the mineral input, reacts with CO₂ to give calcium carbonate (CaCo₃) and silicon dioxide (SiO₂) within the reactor of the disclosed embodiment.

CaSiO₃ + CO₂ → CaCO₃ + SiO₂ equation (1)

The reaction is much more effective in the aqueous phase. When the water H₂O absorbs the CO₂, the aqueous phase becomes acidic with the following species existing simultaneously, given by:

CO₂ + H₂O → H₂CO₃ equation (2)

Furthermore,

H₂CO₃ →← H⁺ + HCO⁻₃ equation (3)

HCO⁻₃ →← H⁺ + CO₃²⁻ equation (4)

H₂O →← H⁺ + OH equation (5)

Then, the calcium contained in the wollastonite dissolves into the aqueous phase as follows:

CaSiO₃ + 2H⁺ → Ca2⁺ + SiO₂↓ + H₂O equation (6)

Ca²⁺+ CO₃²⁻ →← CaCO₃↓ equation (7)

The resident time for the reaction to occur in this example is thirty minutes to six hours and the particle size of the Wollastonite referenced is 38µ (microns). Active mixing is 1500 revolutions per minute (rpm). Temperature maximum is 400°F. Internal bio-reactor pressure in approximately 400 pound per square inch (psi) or 30 bar of P_{CO2} partial pressure.

In the next example 2, UV-C dosing is used. The natural photoexcitation (hv), production of hydroxyls (OH) under atmospheric operating conditions in the upper atmosphere, is artificially re-produced with UV-C ≈ (λ160nm -172nm) in the bioreactor of the disclosed embodiment. M is a collider gas typically Nitrogen.

The following general equations describe the photo-chemical reaction,

O₃ + hv→←O₂ + O(³P) equation (8)

O(³P) + O₂ (+M) → O₃ (+M) equation (9)

O(³P) + H₂O→2OH equation (10)

Compressed air and O₃, which is a by-product, of UV-C photolysis, can be injected into the bioreactor of the disclosed embodiment to supplement hydroxyl (OH) formation, giving,

CaSiO₃+2H⁺ +OH + hv → Ca(OH)₂+SiO₂₍ₛ₎ + H⁺ equation (11)

CO₂ is then injected into the reactor, which gives,

CO₂+Ca(OH)₂+SiO₂₍ₛ₎ → CaCO₃+H₂O+SiO₂(_{aq}) equation (12)

The hydrated slurry is removed from the bioreactor and dried at temperature T ≈ 212°F. Water vapor H₂O _{(aq.- vapor)} is collected, purified, and recycled for later use, which gives,

CaCO₃+ H₂O + SiO_{2(aq)}+ ΔH → CaCO₃+SiO₂ equation (13)

The resident time in example 2 is estimated to be five minutes to one hour, with the particle size of the Wollastonite referenced at 38_{µ} (microns). Temperature is 250°F/400°F. Pressure conditions may vary from 200-400 pounds per square inch (psi) of P_{CO2} partial pressure. Active mixing is 500 rpm to 1500 rpm.

The disclosed reactor incorporates energy recovery devices that include, but are not limited to, ring stator magnets coupled to the blade tips of the propellers and/or motor shaft in different configurations, that can generate DC power when the turbine blades are rotating; compressed CO₂ gas jets directed at shaft mounted turbines and such shaft is also coupled to an electric generator, whereas high pressure fluid energy from CO₂ gas jets translate to mechanical torque of the multi-element turbines. In addition, high pressure steam and compressed air may also be injected and re-injected into the reactor to induce turbine rotation and if the rotating turbine shaft is also coupled to an electric motor, direct current electricity is produced. Thermo electric generators, abbreviated TEG's, are solid state devices using the Seebeck effect to create an electric charge from heat flux. TEG's are integrated into the reactor design. Creating electricity in the form of DC and/or AC power offsets the energy consumption required to operate the disclosed reactor at optimum heat and pressure conditions necessary for carbonation and other type reactions.

The bioreactor of the present embodiment due to its wide functioning range of temperature, pressure and vacuum, mixing, artificial UV, and visible light, also has further application in the production of foods, specifically, cell cultured foods like fish, meats and certain vegetables and plants for human and animal consumption. The reactor volume, material, and design for both inorganic and organic product can be scaled from .014 cubic meters to 150 cubic meters of volume for processing and culturing tissue and organic matter for cell cultured foods in large batches.

The preferred reactor materials are stainless steel and or aluminum. Other light metal alloys, glass, and sapphire attachments may be preferred to some type of plastics for scaling the volume of the reactor. Plastics selection must be done with diligence, due to the risk of transfer of PFAS/PFOA/PFOS which are acronyms for forever chemicals, and potentially toxic chemicals which may exist in plastic products and potentially transfer to the cultured batches incubating in the reactor. However, certain plastics, molded carbonates and polycarbonates that are PFAS/PFOA/PFOS free can be used as material for the reactor lining body. State of the art bioreactors incorporating Teflon material, which is made from PFAS/PFOA/PFOS risk transfer of the group of forever chemicals into the cultured cell organic products, such as meats, fish, and plant products, risking human and non-human ingestion of forever chemicals from transference.

The production of muscle cells for cell cultured meats can be separated into two phases: a proliferation phase where the cells divide and multiply, and a differentiation phase where cells differentiate into skeletal muscle cells, then fuse into multinucleated myotubes. Both phases require different culture environments. During the differentiation phase, a 3D support structure might be required so the skeletal muscle cells can form muscle tissue.

Compared to current state of the art bioreactors, the bioreactor of the disclosed embodiment can diffuse nutrient flow equally throughout the volume and length of the reactor, maintain zonal heating or section heating and cooling, as well as distribute heating, evenly through the cultures in the reactor through variable speed mixing, reverse flow mixing and heat exchange from gas injection.

An ultraviolet (UV) light emitting source and/or a visible light emitting source can be used to irradiate (dose) each tissue culture enclosed within the bioreactor using fiber optic cables, or directly from UV or visible light sources housed withing the cavity of the bioreactor or circumferentially mounted along the outer walls of the bioreactor. UV and visible light dosing can enhance certain cell culture rate of development and is practical for bio photonic sequencing of various cell tissue cultured meats, poultry, fish, certain plants, and vegetables.

The primary method of enhancing cell cultured tissue rate of development from bio photonics is in the germicidal benefit to the nutrient flow and cell tissue cultures incubating in the bioreactor. Viruses, microbial bacteria, (yeast, fungi), and mycoplasma, can cause cell contamination resulting in the loss of cell tissue, and potentially contamination of the complete batch housed in the reactor. UV-C/B/A light is a potent germicidal agent and RNA disruptor to bacteria, viruses, and pathogens, preventing bacterial and viral colony spread in the tissue cultures.

The bioreactor of the present embodiment has multiple ports for injection of reagents, nutrients, gases, and exhaust ports for the removal of slurry waste and cell waste by-products. UV lighting can be used to purify and recycle water that is used for the nutrient broth.

The disclosed next-generation, photolytic reactor with energy recovery capability operates differently than previous, vertical mount type reactors found in laboratories, institutions, and research facilities. The bioreactor design utilizes a multi-element axial propeller agitator in combination with ultra-violet/ UV-A/B LED, liquid emitting diodes and excimer lamps, in the wavelength of 200-400 nanometers, nm, as well as UV-C directed light energy, between 140nm to 200nm., to speed up the time to carbonate CO₂ with magnesium and calcium silicate minerals as well as other mineral waste, oxide feedstocks and desulfurized calcium sulfates. The resultant effect of photolysis, high temperatures, and pressure is the enhanced carbonation of calcium and magnesium silicates in the presence of CO₂ as described in Example 2 in Background Description of the preferred embodiment.

The carbonate precipitate that is produced can be but is not limited to, magnesium carbonate (MgCO₃), calcium carbonate (CaCO₃), dolomitic carbonates ([Mg/Ca]CO₃), iron carbonate, (FeCO₃), potassium carbonates (K₂CO₃), and sodium carbonates (Na₂CO₃). The carbonates then become feedstock materials for a plethora of industrial processes and end use products. The present embodiment consists of reactor pressure vessel housing, cap ends and quartz viewports that can be pressurized up to 2200 psi, pounds per square inch, or 150 atm, atmospheres, to emulate conditions in Earth's upper mantle. The reactor environment can also be de-pressurized to emulate low atmospheric conditions, and vacuum conditions.

The disclosed reactor will also withstand heat up to 2000°F under ambient conditions; up to 625°F under high pressure conditions, and freezing temperature to negative, -20°F. Heating element(s), internal and externally mounted, provide thermal energy for high heat environment simulations. In industrial or plant exhaust settings, the heat from the flue gas exhaust compliments integrated bioreactor heating elements and may result in lower thermal energy consumption. Multi-element mixers agitate exponentially more of the slurry surface area relative to a single stage agitator, commonly used, which will help increase time to precipitation at high temperatures.

During precipitation from mineral carbonation, a passivating silicone layer or PL have been found to form, from past research and experiments, and re-form during the carbonation process, which inhibits the time to precipitate carbonates. In natural weathering of mineral and rock formations, laterite layers occur which are weathered surface areas. Laterite strata prevent or inhibit the un-weathered mineral surface area beneath it from weathering, unless the layer is fractured, exposed, or removed. The multi-element mixers disclosed operates up to 1500rpm to persistently chip away and fracture the PL, passivating layer that forms and re-forms at high temperatures and pressure during the carbonation process.

UV-C photolysis induces hydroxy radical (OH), which in turn can bind with leached calcium and magnesium silicates to create hydroxides that react with injected CO₂ to then create carbonates in an aqueous slurry that may contain additives or admixtures. Artificial UV-C light emitters disclosed as part of the reactor, emulate photo dissociation of Oxygen when exposed to UV-C from sunlight, like ozone creation, in the upper atmosphere. Photolysis, also called photo dissociation and photo decomposition, is a chemical reaction in which an inorganic chemical or an organic chemical is broken down by energetic photons and is the interaction of one or more photons with one target molecule. By using photolysis with UV-C directed, light, reaction times per reactor batch of mineral feedstocks and CO₂ are significantly enhanced. Photolysis can potentially reduce carbonation times to 1 hours or less. Throughout the embodiment, the reference to UV or visible light radiation or dosing is limited to non-ionizing wavelengths of UV and visible light waves.

Further, the disclosed reactor recovers energy consumed during the reaction process through implementation of blade tip ring permanent magnet generators. The mixer turbine acts as the rotor of a generator and is enclosed by stator ring housing with windings to complete the generator configuration. Additionally, pressure energy recovery systems are used as part of the disclosed reactor design that transfer and re-inject waste pressure during and post reaction, to offset energy inputs required for operating the reactor system.

Figure 1A depicts the photolytic reactor, components, and sub-assemblies. The bioreactor system is divided into 3 major functional segments A, B, and C. Depicted in Figure 1A are boundary line separators that delineate between the three major functional sections A, B, C of the bioreactor system. Section A boundary begins with the electric motor housing 1 and terminates at the straight pipe cap end 19 and flange 2. Section B begins at the end of Section A and terminates at the split retaining wall cap end flange 6. Section C begins at the end of Section B and terminates at the exhaust tube 15. Section A is the Power Drive Section, Section B is the Venturi Nozzle Section, and Section C is the UV and Visible Light Section. Each section is modular. Within Section A, an electric motor is enclosed by the electric motor housing 1.

The electric motor within the housing cover 1 is mounted to a frame stand 9, which supports the electric motor. Alternate facing flange pairs 2 and 6, typically referred to as male - female slip-on flanges are shown. The straight section pipe cap end 19, is fastened by weldment to flanges 2. Flanges 2 are then joined together by high strength, high temperature fasteners 2.1. In Section B, the venturi nozzle 11, 12, 12.1, and 13 form the central pressure body of the bioreactor system of FIG. 1A. The venturi nozzle geometry of Section B facilitates increased improvement in fluid flow compared to a continuous straight cylinder or tube. The venturi nozzle section 12 diverges to a straight section 11. The venturi nozzle section 12.1 diverges to a short straight section tube 13. The straight section tubes 11, and 13 of Section B are attached by weldment to the alternate facing flanges 2 and flanges 6. Welded to the back face of the flanges 6 is a cap end tube with split retaining walls which comprises a component of Section C in Section C, the cap end tube with split retaining walls is enclosed by a UV nacelle -shield fairing 8. Attached to the UV nacelle-shield fairing 8 is an exhaust nozzle 15 connected to a sleeved support tube 14 that facilitates 360° rotation of the exhaust tube 15. The UV nacelle - shield fairing 8 is a modular section that can be detached or secured to the split retaining wall cap end tube by clamp 7.

The sleeved support tube 14 allows the exhaust nozzle 15 to rotate 360°, degrees, to direct ozone waste into water tanks or storage bins of different stack height. Ozone, chemical formula, O₃, is generated by the dissociation of ambient Oxygen, chemical formula O₂, from high intensity, UV-C LED, light emitting diodes, and lamps during batch reaction. The UV nacelle-shield fairing 8 is also a barrier shield to UV-C light and eliminates any chance of human exposure to UV-C light energy when installed. Two ports 3, 4, allow for media feedstock and CO₂ gas to be injected into Section B, then the gas diffuses through Sections A, B and can be exhausted from Section B of the bioreactor system. A heating element or heating band 5 controls the thermal environment of the reactor assembly in FIG. 1A. The heating band 5 is installed around the external surface diameter of the convergent section 12.3 (see FIG 2C and FIG 3B and FIG 3D and FIG 6) or constricted section of the venturi nozzle in Section B. The reactor body and assembly of FIG. 1A is supported by two block stands 10 of Section A, and 10.1 of Section B.

FIG. 1B is an ISO or isometric right-side view of the bioreactor assembly of FIG. 1A without the fairing cover 1, FIG. 1A with 3D detail of the electric motor 22, electric motor shaft 22.1, driveshaft coupling 22.2, main drive shaft 21, flanged cap ends 2, FIG. 1A, 2 and straight section pipe cap end 19, FIG. 1A. 19, split retaining wall flanged cap ends 6, FIG. 1A, 6, heating bands-elements 5, FIG. 1A, 5, and distant view of the UV nacelle-shield fairing 8, FIG. 1A, 8, and exhaust nozzle 15, FIG. 1A, 15 configurations. During operation, the electric motor 22, rotates the electric motor shaft 22.1 that is fixedly connected to a shaft coupling 22.2, which is fixedly connected to the main drive shaft 21, which may or may not be of the same diameter of the electric motor shaft 22.1. Rotation can occur at variable speeds from 1 - 1800 revolutions per minute under ambient pressure and at gas mix or CO₂ partial pressure up to 2200 pounds per square inch, psi or in vacuum environment approaching -5 psi or -517 torr.

The rotation of the main drive shaft 21, imparts rotation on the mixer-agitator turbines, which agitates, mixes and disperses the slurry and/or media, and any other liquid, solid or gaseous feedstock, organic or inorganic, that is in the reactor. The term slurry is defined as a mixture of inorganic mineral feedstock of various particle sizes, combined with water and additives. The slurry may be pre-mixed and injected into the reactor or the slurry may be created in the reactor from separate injection of water, additives, and inorganic mineral feedstock.

Through mixer rotation, heat is evenly distributed throughout sections A, B, and C of FIG. 1A and the bioreactor assembly of FIG 1B, because of the Venturi affect and placement of electric band heaters or electric heating elements 5, FIG. 1A, 5, on the exterior surface of the constricted section of the venturi nozzle 12, 12.1, FIG. 1A. Section B. The exhaust nozzle 15, FIG. 1A, sleeved support tube 14, FIG.1A, and additional slurry injection port 4.1 are shown in relative position to the UV nacelle-shield fairing 8, FIG. 1A. Additional injector port(s) 34, are for gases, inorganic slurries port 35, organic cultures port 36, nutrients for organic cultures and general additives port 37 are shown mounted along the surface of the venturi nozzle section 12, FIG. 1A.

Figure 1C is an isometric and enlarged view of the electric motor 22, FIG, 1B, without the motor fairing - housing 1, FIG. 1A, and depicting the shaft coupling 22.2, FIG. 1B, main drive shaft 21, FIG. 1B, at least one seal-bearing shaft support assembly 20, at least one retaining shaft support plate 20.1, fasteners 41, and cap end flange 2, FIG. 1A, FIG. 1B, with straight pipe section 19, FIG. 1A, FIG. 1B.

Figure 2A illustrates, in a cross-section view, the mixer agitator axial flow turbines 25, 25.1, 25.2 and 26, 26.1, and 26.2, which is a multi-element configuration for persistent and intermittent mixing of the media under reaction within the venturi reactor body sections 12, 12.1 11, 13, FIG. 1A, FIG. 1C. The main drive shaft 21, FIG. 1B, FIG. 1C, is shown extending horizontally from the shaft coupling 22.2, FIG. 1B, FIG. 1C, passing through a seal-bearing shaft support assembly 20, FIG. 1C, The main drive shaft 21, FIG. 1B, FIG. 1C passes through the venturi nozzle sections 12, 12.1, 11, 13, FIG. 1A, FIG. 1B, and terminates at the venturi nozzle divergent section 13, FIG. 1A. The mixer-agitator axial flow turbines 25, 25.1, 25.2 and 26, 26.1, 26.2 agitate the media under reaction. Enclosed within the split retaining wall cap-end 24 flange 6, FIG. 1B, FIG. 1A, is a crucible 17. A clear translucent material forms the crucible 17 and the clear translucent material allows for transmissivity of UV and visible light emitted from an array of UV LED's, light emitting diodes and visible light emitters 23, and said UV and visible light emitters 23 are circumferentially installed on a plated structure 16 surrounding the split retaining wall cap end 24. Support of the electric motor 2, FIG. 1B, FIG 1C, is provided by a platform support structure 9, FIG. 1A. Section A, B, C of FIG. 1A are depicted for reference.

The UV or visible light emitter 23 can now dose the media under reaction at high or low temperature and high or low pressure, or in a vacuum, to enhance reaction kinetics. An additional pressure tap port 4.1, allows high pressure CO₂ and steam to be injected onto the turbine blades inside the venturi nozzle sections 12, 12.1, FIG. 1A, FIG. 1B, to induce rotation of the mixer agitator axial flow turbines, 25, 25.1, 25.2 and 26, 26.1, 26.2. which rotates the electric motor 22, FIG. 1B, FIG 1C, 22 Re-injecting CO₂ and steam partially offsets the energy inputs from operating the bioreactor system of FIG. 1A, FIG. 1B, FIG. 1C, FIG. 2A.

The band heater or heating element 5, FIG. 1A, FIG 1B, is the primary heating source to control the thermal environment inside the reactor body of FIG. 1A, Sections A, B, and C, FIG. 1B, and FIG. 2A. Once heat is distributed through-out the core of the bioreactor assembly of FIG. 1A, Sections A, B, C and optimal reaction temperature is reached, CO₂ and other inert gases can be injected through port(s) 4, 4.1, in FIG. 1A and FIG 1B, and then enters the venturi nozzle reactor cavity shown in FIG. 1A, FIG. 1B, FIG. 2A, in a super-critical and/or critical state depending on the thermal gradient set by the heat element 5 shown in FIG. 1A and FIG. 1B, and partial pressure of CO₂ at the time of inj ection.

Figure. 2B is a left side perspective view of the complete bioreactor assembly of FIG. 1A, FIG. 1B and FIG. 2A without the electric motor housing cover 1 of FIG. 1A. The UV nacelle-shield fairing 8, FIG. 1B, FIG. 1A, is shown with exhaust nozzle 15 in FIG. 1A and FIG. 1B. The flanges 6 and 2 in FIG. 1A, FIG. 1B and FIG. 2A are depicted in a left side perspective view. Port(s) 4.1 shown in FIG. 1A and FIG. 2A, and additional port(s) 30 are illustrated. The electric motor 22 shown in FIG. 1B, FIG. 1C and FIG. 2A, and shaft coupling 22.2 shown in FIG. 1B, FIG. 1C and FIG. 2A are shown in relative position to the UV nacelle-shield fairing 8.

Figure 2C is an oblique drawing of the left side of the bioreactor beginning at the main drive shaft 21 shown in FIG. 1B, FIG. 1C and FIG. 2A and ending at the UV nacelle-shield fairing 8 shown in FIG. 1A, FIG. 1B and FIG. 2B. A cross-section, oblique view of the venturi nozzle sections 12, 12.1 and 11 shown in FIG. 1A. FIG. 1B, FIG. 2A reveals the mixer-agitator axial flow turbines 26.2, 26, 25, 25.2, FIG. 2A oriented along the main drive shaft 21 shown in FIG. 1B, FIG. 1C and FIG. 2A. Two perforated conical cylinder 33, 33.1 are shown mounted to the interior walls of the venturi nozzle segments 12, 12.1 shown in FIG. 1A. FIG. 1B and FIG. 2A. The primary purpose of the perforated cylinders 33, 33.1 are to house inorganic and organic feedstock separately from the rest of the constituents of the reactor cavity.

In the case of organic feedstocks, like cell tissue cultures, pre-loading the perforated cylinders 33, 33.1 with the cell tissue organic culture could be preferred to injection of the cell tissue culture. Each cylinder 33, 33.1 perforations allow for organic and inorganic nutrients injected into the reactor body cavity of FIG. 1A, FIG. 1B. FIG. 2A, FIG. 2B to flow into and exit from the cylinders 33, 33.1. An additional port 31 is also shown.

FIG. 2D is an oblique drawing of the left side of the bioreactor system of FIG. 1A, FIG. 1B, FIG. 2A, FIG. 2B, FIG. 2C. Depicted in a cross-section view is the interior cavity of the straight pipe cap end 19, FIG. 1A, FIG. 1C, FIG. 2A, FIG. 2C, The main drive shaft 21, FIG. 1B, FIG. 1C, FIG. 2A, FIG. 2C is shown enclosed by at least 3 sealing glands 20.2 for pressurization. The cross-section of at least one bearing-seal support housing 20, FIG. 1C, FIG. 2C, and at least one retaining support plate 20.1, FIG. 1C, FIG. 2C for support of the main drive shaft 21, FIG. 1B, FIG. 1C, FIG. 2A, FIG. 2C is shown. A cross-section view of one of the flange pair 2, FIG. 1A, FIG. 1B, FIG. 1C, FIG. 2A, FIG. 2B, FIG. 2C is also shown.

Figure 3A is a close, perspective view of the UV and visible light, LED, light emitting diodes emitters 23 and plate mount structure 16, FIG. 2A, installed around the split retaining wall cap end 24, FIG. 2A. and flange 6, FIG. 1A, FIG. 1B, FIG. 2A, FIG. 2B, FIG. 2C. The UV and visible light LED, light emitting diodes, emitters 23 direct UV and visible light at wavelengths from 120nm-700nm through a clear material that forms a crucible 17, FIG. 2A, to photo dissociate the media and gas content of the reactor assembly of FIG. 1, FIG. 1B, FIG. 2A, FIG. 2B, FIG. 2C, FIG. 2D. The crucible 17, FIG. 2A, is hollow and is of considerable thickness and material to handle pressurization up to 2200 psi.

Multiple UV and visible light-LED, light emitting diodes and lamps 23 are housed and supported by the plate mount structure 16, FIG. 2A. The crucible 17, FIG. 2A, is sealed inside the split retaining wall 24, FIG. 2A, cap end flange 6, FIG. 1A, FIG. 1B, FIG. 2A, FIG. 2B, FIG. 2C. The split retaining wall 24, FIG. 2A, is attached by weldment, screw thread, or other fastening method to the cap end flanges 6, FIG. 1A, FIG. 1B, FIG. 2A, FIG. 2B, FIG. 2C, and attached to or supported by a block mount 10.1, FIG. 1A, FIG. 2A.

Figure 3B is a dimetric view of the split retaining wall cap end 24, FIG. 2A, FIG. 3A. The cross-sectional view of the UV nacelle-shield fairing 8, FIG. 1A, FIG. 1B, FIG. 2B, FIG. 2C reveals the placement and orientation of the UV and visible light source emitters 23, FIG. 3A, and plated mount structure 16, FIG. 3A. The crucible 17, FIG. 2A, FIG. 3A, housed within the split retaining wall cap end 24 FIG. 2A, FIG. 3A, flanges 6, FIG. 1A, FIG. 1B, FIG. 2A, FIG. 2B, FIG. 2C, FIG. 3A is depicted.

Figure 3C is a 2D right side elevation, and cross-section view of the reactor internal cavity of FIG. 1A, FIG. 1B, FIG. 2A, FIG. 2B, FIG. 2C, FIG. 2D, FIG. 3B. Depicted are fiber optics and fiber optic cables 39, 39.1 mounted to the internal walls. The fiber optic cables 39, 39.1 transmit UV and visible light directly into the cavity to bombard more surface area of the slurry under reaction with photons. The UV or visible light source emitter 40 with fiber optic cable 39, 39.1 attachment is mounted outside of the split retaining wall cap end 24, FIG. 2A, FIG. 3A, FIG. 3B.

Figure 3D is a perspective view, with cross-sectional views of the UV nacelle-shield fairing 8, FIG. 1A, FIG. 1B, FIG. 2B, FIG. 2C, FIG. 3B, crucible 17, FIG. 2, FIG. 3A, FIG. 3B, FIG. 3B, and venturi nozzle segments, 12, 12.3, 12.1, 11, FIG. 1, FIG. 1B, FIG. 2A, FIG. 2B, FIG. 2C, FIG. 2D, FIG. 3B, FIG. 3C. The UV and visible light emitter 40 with fiber optic cable 39, 39.1, FIG. 3C, attachments are shown. The fiber optic cables 39, 39.1, FIG. 3C are shown to originate from the emitter 40, which is installed within the split wall retaining cap end 24, FIG. 2A, FIG. 3A, FIG. 3B. FIG. 3C. The fiber optic cables 39, 39.1, FIG. 3C, extend through the crucible 17, FIG. 2A, FIG. 3A, FIG. 3B, FIG. 3C, and end within the bioreactor cavity at venturi nozzle diverging segment 11, FIG. 1A, FIG. 2A, FIG 2D, Illustrated also is the duct passage 43 within the UV nacelle-shield fairing 8, FIG. 1A, FIG. 1B, FIG. 2A.1, FIG. 2C, FIG. 3B.

Figure 4 is a cross-section, close, perspective view of at least one mixer-agitator axial flow turbine 25.2, FIG. 2A, FIG. 2C, with blade tip stator ring with windings 27. This configuration of the rotating mixer-agitator axial flow turbine rotor 25.2, FIG. 2A, FIG. 2C, and stator ring with winding 27, is that of a permanent magnet generator capable of producing DC power when the main drive shaft 21, FIG. 1B, FIG. 1C, FIG. 2A, FIG. 2C, FIG. 2D, FIG. 3D is rotating. The tips of the mixer-agitator axial flow turbine rotor 25.2, FIG. 2A, FIG. 2C, are sealed inside a stator ring with winding 27 and when rotating produce DC current. The exposed mixer-agitator axial flow turbine surface area 25.2, FIG. 2A, FIG. 2C, is used to agitate and mix the media and/or slurry.

Figure 5 highlights the CO₂ gas and other waste gas stream flow through and out of the bioreactor cavity depicted in cross section views in FIG. 2A, FIG. 2C, FIG. 2D, FIG. 3D and the return route. The bold directional arrows represent gas and slurry exiting ports 31, FIG. 3D, FIG. 3C, FIG. 2D, FIG. 2C, FIG. 2B, and additional port(s) 30. The gas-slurry flow first enters a filter 44 that strains and filters the slurry, allowing the bypass gas to circulate out of the filter-strainer 44. During operation, the core of the venturi nozzle sections of FIG. 1A, sections A, B, C, and venturi nozzle sections 11, 12, 12.1, FIG. 1B, FIG. 2A, FIG. 2C, FIG. 2D, FIG. 3C, FIG. 3D, and venturi nozzle section 13, FIG. 1A, and FIG. 2A, is thermally adjusted and held at certain temperature levels over varying time intervals, to facilitate reaction of the media inside the reactor. To offset energy inputs needed for the electric motor, heating, and compression; pressure within the reactor is exhausted through pressure ports 31, FIG. 2B, FIG. 2C, FIG. 2D, FIG. 3C, FIG. 3D, and port 30. The arrows with darkened round ends represent the flow of CO₂ and the exhaust steam outside of the bio-reactor assembly. The CO₂ and exhaust steam pass through a liquid-gas booster pump 28 which increases pressure of the CO₂ and steam. The CO2 and steam are re-injected through additional pressure port 29 and the flow is focused onto a mixer-agitator axial flow turbine 42, FIG. 3D or any other mixer agitator axial flow turbine, 26.2, 26.1, 26, 25, 25.1, 25 of FIG. 2A, FIG. 2C, FIG. 2D, FIG. 3D, to induce rotation of the main drive shaft 21, FIG. 1B, FIG. 1C, FIG. 2A, FIG. 2C, FIG. 2D, FIG. 3D. The torque then rotates the electric motor-generator 22, FIG. 1B, FIG. 1C, FIG. 2A, FIG. 2B, and DC current is produced. The power generated from steam and CO₂ re-injection is now available to be consumed or stored; thereby, potentially offsetting the power input needed to operate the reactor system.

In Figure 6, the venturi nozzle cavity section B of FIG. 1A is depicted in a cross-section cut away view. Housed within the cavity segment B of FIG. 1A, are at least 2 perforated containers 33, 33.1, FIG. 2C, FIG. 2D, FIG. 3D, that can store inorganic and organic media, like mineral feedstock, if inorganic material is used, or if the application is for cell cultured foods, the perforated containers 33, 33.1, FIG. 2C, FIG. 2D, FIG. 3D can house cell tissue cultures. The perforated containers 33. 33.1, FIG. 2C, FIG. 2D, FIG. 3D can be mounted to the interior walls of the venturi reactor cavity of segment B of FIG. 1A, allowing the main drive shaft 21, FIG. 1B, FIG. 1C, FIG. 2A, FIG. 2C, FIG. 2D, FIG. 3D, FIG. 4 to pass through the bore section of the perforated containers 33, 33.1, FIG. 2C, FIG. 2D, FIG. 3D The attachment method previously described can also facilitate dispersion of heat, cooling, gas, liquid, mineral and nutrient flow through the organic or inorganic media housed within the perforated containers 33, 33.1, FIG. 2C, FIG. 2D, FIG. 3D.

The mixer-agitator axial flow turbines 26.2, 26.1, 25.1, 25.2, FIG. 2A, FIG. 2C, FIG. 2D, are mounted to the main drive shaft 21, FIG. 1B, FIG. 1C, FIG. 2A, FIG. 2C, FIG. 2D, FIG. 3D, FIG. 4 and provide mixing flow and dispersion of inorganic media or organic media, like a nutrient broth, which may be solids, liquids, and gases or combination thereof. The rotation of the mixer-agitator axial flow turbines 26.2, 26.1, 25.1, 25.2, FIG. 2A, FIG. 2C, FIG. 2D, evenly distribute heat and cooling fluid flows to the inorganic or organic media throughout the reactor cavity and to inorganic and organic media such as cell tissue cultures, housed in the perforated containers 33, 33.1, FIG. 2C, FIG. 2D, FIG. 3D. at various revolutions per minute (rpm). Figure 6 denotes port(s) 34, 35, 36, 37, FIG. 1B and additional port 38 mounted to the outside walls of the venturi nozzle reactor segment 12, FIG. 1A, FIG. 1B, FIG. 2A, FIG. 2C, FIG. 2D, FIG. 3B, FIG. 3C, FIG. 3D, FIG. 5,

Each port(s) 34, 35, 36, 37, FIG. 1B, and 38 can be arbitrarily designated. For example, through ports 34, FIG. 1B, gas is injected, port(s) 35, FIG. 1B, for mineral feedstock, or liquids through port 36, FIG. 1B, or solids through port 37, FIG. 1B, and inorganic or organic slurry injection through additional port 38. Injection of liquid, solids, and gases facilitate a contactless feedstock loading method, to minimize and prevent contaminant disruption to the carbonation or nucleation of inorganic mineral feedstock with CO₂ gas and multi-nucleation of organic feedstock, primarily the tissue cell culture housed within the perforated containers 33, 33.1, FIG. 2C, FIG. 2D, FIG. 3D.

The heating unit or heat element 5, FIG. 1A, FIG. 1B, FIG. 2A, FIG. 2B, FIG. 2C, FIG. 2D, FIG. 3D, depicted as a trapezoid, produces waste heat flow, denoted by directional bold arrows, which is then conducted via heat-exchange, thermocouple, or other type of heat conducting material to thermal electric generators 32 of FIG. 6. The thermoelectric generators or TEG's 32, are energy recovery devices, that utilizes the heat flux from the reactor outer surface of segments A, B and C of FIG. 1A and the heating band-element, 5, FIG. 1A, FIG. 1B, FIG. 2A, FIG. 2B, FIG. 2C, FIG. 2D, FIG. 3D, to generate electricity via the Seebeck effect.

Figure 7 is a perspective, close view of only the perforated cylinders 33, 31, FIG. 2C, FIG. 2D, FIG. 3D. oriented along the main drive shaft 21, FIG. 1B, FIG. 1C, FIG. 2A, FIG. 2C, FIG. 2D, FIG. 3D, FIG. 4, FIG. 6 The mixer-agitator axial flow turbines 26.2, 26.1, 25.1, 25.2, FIG. 2A, FIG. 2C, FIG. 2D, FIG. 6 rotate in any direction at variable rpm for the purpose of mixing, mechanical activation pre-treatment, media surface agitation, heating, cooling, fluid and media dispersion of liquids, solids and gases under reaction.

Figure 8 is a dimetric view of the of the multi-port configurations referenced throughout the description of the present embodiment. Illustrated are the ports in the Venturi Nozzle Module of Section B, FIG. 1A and FIG. 2A. The port configuration, location, and number of ports may vary based on the application and media type injected into the Venturi Nozzle Module, Section B of FIG. 1A and FIG. 2A. Pictured are two heating bands or heating elements 5, FIG. 1A, FIG. 2A, FIG. 2B, FIG. 2C, FIG. 2D, FIG. 3D, and FIG. 6, mounted circumferentially on the convergent section outer walls 12.3, FIG. 2C, FIG. 3B, FIG. 3C , FIG. 6 of the Venturi Nozzle Module, Section B of FIG. 1A and FIG. 2A. Ports 34,35,36,37,38, FIG.6 are shown in spatial reference to ports 31, FIG. 5, FIG. 3C, FIG.2D, FIG. 2C, FIG. 2B, port 30, FIG. 5, port 4.1, FIG. 1B, FIG. 2A, FIG. 2B, FIG.2C, FIG.2D, FIG. 3B, FIG. 3C, FIG. 3D, FIG.6, port 4, FIG. 1A, FIG. 2A, port 3, FIG. 1A, and port 29, FIG. 5

The foregoing detailed description of the exemplary and preferred embodiments is presented for purposes of illustration and disclosure in accordance with the requirements of the law. It is not intended to be exhaustive nor to limit the invention to the precise form or forms described, but only to enable others skilled in the art to understand how the invention may be suited for a particular use of implementation. The possibility of modifications and variations will be apparent to practitioners skilled in the art. No limitation is intended by the description of exemplary embodiments which may have included tolerances, feature dimensions, specific operating conditions, engineering specifications, or the like and which may vary between implementations or with changes to the state of the art, and no limitation should be implied therefrom. This disclosure has been made with respect to the current state of the art but also contemplates advancements and that adaptations in the future may take into consideration of those advancements namely in accordance with the then current state of the art.

## Claims

1. A photolytic bioreactor system to convert CO₂ by reaction with CO₂ reactive feedstocks to produce carbonate reaction materials comprising:
a venturi nozzle section comprising:
a central pressure body portion, the central pressure body portion comprising a convergent divergent venturi nozzle extending along a horizontal axis;
a heating band installed around an external surface diameter of a convergent section of the convergent divergent venturi nozzle;
a mixer agitator assembly comprising a plurality of axial flow turbines installed along the horizontal axis on a drive shaft for selectable rotation within the convergent divergent venturi nozzle of an axial flow aqueous slurry having an initial composite comprising the CO₂ reactive feedstocks and input slurry, the mixer agitator assembly being rotatable in clockwise and counterclockwise direction upon selection by a power module portion comprising a direction selectable electric motor connected to drive the drive shaft;
at least one port entry into the convergent divergent venturi nozzle to provide entry flow of CO₂ into the convergent divergent venturi nozzle;
at least one port entry into the convergent divergent venturi nozzle to provide entry flow of the initial slurry into the convergent divergent venturi nozzle;
a light section comprising:
a crucible formed of light translucent wall and the section having at least one light emitter source connected to an open end of the convergent divergent venturi nozzle whereby the axial flow aqueous slurry flows into the crucible and the at least one light emitter source is directed into the crucible to dose the CO₂ reactive feedstocks of the axial flow aqueous slurry into reaction to create carbonate product;
a power module section comprising:
the drive shaft extending from the convergent divergent venturi nozzle section to the power module section;
a reversible motor connected to the drive shaft being selectively operable for selected direction of rotation;
whereby upon operation of the system, entering CO₂ reactively intermixes with the initial slurry to continuously cause reaction to result in carbonate reaction materials as a product of the feedstock.

2. The photolytic bioreactor of claim 1, wherein the at least one light emitter source comprises an untraviolet light emitter source.

3. The photolytic bioreactor of claim 1, wherein the at least one light emitter source comprises a visible light emitter source.

4. The photolytic bioreactor of claim 1 further comprising organic feedstocks within the axial flow aqueous slurry are enclosed within at least one perforated cylinder housed within the reactor, for multi-nucleation of cell tissue cultures.

5. The photolytic bioreactor of claim 1 whereas at least one fiber optic strand and at least one fiber optic cable is mounted along the interior walls of the bioreactor for the purpose of dosing organic feedstock, organic and inorganic media, inorganic and organic components of the axial flow aqueous slurry under reaction in the bioreactor.

6. The photolytic bioreactor of claim 1 also comprising at least one thermo-electric generator for converting waste heat to electricity,

7. The photolytic bioreactor of claim 1 also comprising at least one permanent magnet generator enclosed around the axial flow turbines for the purpose of generating electricity to offset the energy consumed from operating the reactor,

8. The photolytic bioreactor of claim 1 wherein the light section further comprises a visible light emitting source to dose selected feedstocks of the axial flow aqueous slurry into reaction.

9. The photolytic bioreactor of claim 1 wherein the source of CO₂ is at least from industrial emissions.

10. The photolytic bioreactor of claim 1 wherein the source of CO₂ is at least from industrial emissions and direct air captured CO₂.

11. A method for producing carbonate reactive materials comprising:
collecting an input slurry comprising an initial slurry and CO₂ ; and
processing the collected input slurry in a convergent divergent venturi nozzle section of a bioreactor, the bioreactor comprising:
a central pressure body portion, the central pressure body portion comprising a convergent divergent venturi nozzle extending along a horizontal axis;
a heating band installed around an external surface diameter of a convergent section of the convergent divergent venturi nozzle;
a mixer agitator assembly comprising a plurality of axial flow turbines installed along the horizontal axis on a drive shaft for selectable rotation within the convergent divergent venturi nozzle of an axial flow aqueous slurry having an initial composite comprising the CO₂ reactive feedstocks and input slurry, the mixer agitator assembly being rotatable in clockwise and counterclockwise direction upon selection by a power module portion comprising a direction selectable electric motor connected to drive the drive shaft;
at least one port entry into the convergent divergent venturi nozzle to provide entry flow of CO₂ into the convergent divergent venturi nozzle;
at least one port entry into the convergent divergent venturi nozzle to provide entry flow of the input slurry form into the convergent divergent venturi nozzle;
looping the axial flow aqueous slurry back into the convergent divergent venturi nozzle section by engaging the mixer agitator to rotate selectively clockwise and counterclockwise;
processing the output of the convergent divergent venturi in a light section comprising:
a crucible of translucent light transmissible material connected to an open end of the convergent divergent venturi nozzle whereby the axial flow aqueous slurry flows into the crucible and at least one light emitter source directed into the crucible to dose the CO₂ reactive feedstocks of the axial flow aqueous slurry into reaction to create carbonate product;
whereby upon operation of the system, entering CO₂ reactively intermixes with the axial flow aqueous slurry to continuously cause reaction to result in carbonate reaction materials as a product of the feedstock.

12. The method of Claim 11 wherein the organic feedstocks within the axial flow aqueous slurry are enclosed within at least one perforated cylinder housed within the bioreactor, for multi-nucleation of cell tissue cultures.

13. The method of Claim 11 wherein at least one fiber optic strand and at least one fiber optic cable is mounted along the interior walls of the bioreactor for the purpose of dosing organic feedstock, organic and inorganic media, inorganic and organic compoients of the axial flow aqueous slurry under reaction in the bioreactor.

14. The method of Claim 11 wherein the bioreactor also comprises at least one thermo-electric generator for converting waste heat to electricity,

15. The method of Claim 11 wherein the bioreactor also comprises at least one permanent magnet generator enclosed around the axial flow turbines for the purpose of generating electricity to offset the energy consumed from operating the reactor,

## Patentansprüche

1. Photolytisches Bioreaktorsystem zum Umwandeln CO₂ durch Reaktion mit CO₂-reaktiven Ausgangsstoffen zur Herstellung von Karbonat-Reaktionsmaterialien, umfassend:
einen Venturidüsenabschnitt, umfassend:
einen zentralen Druckkörperabschnitt, wobei der zentrale Druckkörperabschnitt eine konvergente divergente Venturidüse umfasst, die sich entlang einer Horizontalachse erstreckt;
ein Heizband, das um einen Außenflächendurchmesser einer konvergenten Abschnitt der konvergenten divergenten Venturidüse installiert wird;
eine Mischer-Rührwerksanordnung, umfassend eine Vielzahl von Axialströmungsturbinen, die entlang der Horizontalachse auf einer Antriebswelle installiert werden, zur wählbaren Drehung innerhalb der konvergenten divergenten Venturidüse eines axialströmenden wässrigen Schlammes aufweisend eine Anfangszusammensetzung, die die CO2-reaktiven Ausgangsstoffe und den Eingangsschlamm umfasst, wobei die Mischer-Rührwerksanordnung bei Auswahl durch einen Leistungsmodulabschnitt umfassend einen richtungswählbaren Elektromotor in Uhrzeiger- und Gegenuhrzeigerrichtung drehbar ist, wobei der Elektromotor verbunden ist, um die Antriebswelle zu betätigen;
mindestens einen Öffnungseintritt in die konvergente divergente Venturidüse, um eine Eintrittsströmung von CO2 in die konvergent-divergente Venturidüse bereitzustellen;
mindestens einen Öffnungseintritt in die konvergente divergente Venturidüse, um eine Eintrittsströmung des Anfangsschlamms in die konvergente divergente Venturidüse bereitzustellen;
einen Lichtabschnitt, umfassend:
einen Tiegel, der aus lichtdurchlässiger Wand ausgebildet wird und der Abschnitt aufweisend mindestens eine Lichtsenderquelle, die mit einem offenen Ende der konvergenten divergenten Venturidüse verbunden ist, wodurch der axial strömende wässrige Schlamm in den Tiegel fließt und die mindestens eine Lichtsenderquelle in den Tiegel gerichtet ist, um die CO2-reaktiven Ausgangsstoffe des axial strömenden wässrigen Schlammes in Reaktion zu dosieren, um ein Karbonatprodukt herzustellen;
einen Leistungsmodulabschnitt, umfassend:
die Antriebswelle, die sich von dem Venturidüseabschnitt bis zu dem Leistungsmodulabschnitt erstreckt;
einen reversiblen Motor, der mit der Antriebswelle verbunden ist und selektiv für eine ausgewählte Drehrichtung betrieben werden kann;
wodurch beim Betrieb des Systems, eintretendes CO₂, reaktiv mit dem Anfangsschlamm vermischt wird, um kontinuierlich eine Reaktion zu bewirken, die Karbonat-Reaktionsmaterialien als ein Produkt der Ausgangsstoffe zum Resultat hat.

2. Photolytischer Bioreaktor nach Anspruch 1, wobei die mindestens eine Lichtsenderquelle eine ultraviolette Lichtsenderquelle umfasst.

3. Photolytischer Bioreaktor nach Anspruch 1, wobei die mindestens eine Lichtsenderquelle eine sichtbare Lichtsenderquelle umfasst.

4. Photolytischer Bioreaktor nach Anspruch 1 ferner umfassend organische Ausgangsstoffe innerhalb des axial strömenden wässrigen Schlammes, die in mindestens einem perforierten Zylinder eingeschlossen sind, der in dem Reaktor aufgenommen wird, zur Multi-Nukleation von Zellgewebekulturen.

5. Photolytischer Bioreaktor nach Anspruch 1, wobei mindestens ein Lichtwellenleiter-Strang und mindestens ein Lichtwellenleiterkabel entlang der Innenwände des Bioreaktors montiert sind, um organische Ausgangsstoffen, organische und anorganische Medien, anorganischen und organische Komponenten des axial strömenden wässrigen Schlammes unter Reaktion in dem Bioreaktor zu dosieren.

6. Photolytischer Bioreaktor nach Anspruch 1, ferner umfassend mindestens einen thermoelektrischen Generator zum Umwandeln Abwärme in Elektrizität.

7. Photolytischer Bioreaktor nach Anspruch 1 ferner umfassend mindestens einen Permanentmagneten-Generator, der um die Axialströmungsturbinen angeordnet ist, um Elektrizität zu erzeugen, um den Energieverbrauch durch den Betrieb des Reaktors auszugleichen.

8. Photolytischer Bioreaktor nach Anspruch 1, wobei der Lichtabschnitt ferner eine sichtbare Lichtsenderquelle umfasst, um ausgewählte Ausgangsstoffe des axial strömenden wässrigen Schlammes in Reaktion zu dosieren.

9. Photolytischer Bioreaktor nach Anspruch 1, wobei die CO₂-Quelle mindestens aus industriellen Emissionen stammt.

10. Photolytischer Bioreaktor nach Anspruch 1, wobei die CO₂-Quelle mindestens aus industriellen Emissionen und aus CO₂ stammt, das direkt von Luft eingefangen wird.

11. Verfahren zur Herstellung von karbonatreaktiven Materialien, umfassend:
- Sammeln eines Eintrittsschlammes umfassend einen Anfangsschlamm und CO₂; und
- Verarbeiten des gesammelten Eintrittsschlammes in einem konvergenten divergenten Venturidüsenabschnitt eines Bioreaktors, wobei der Bioreaktor umfasst:
einen zentralen Druckkörperabschnitt, wobei der zentrale Druckkörperabschnitt eine konvergente divergente Venturidüse umfasst, die sich entlang einer Horizontalachse erstreckt;
ein Heizband, das um einen Außenflächendurchmesser eines konvergenten Abschnitts der konvergenten divergenten Venturidüse installiert ist;
eine Mischer-Rührwerksanordnung umfassend eine Vielzahl von Axialströmungsturbinen, die entlang der Horizontalachse auf einer Antriebswelle installiert werden, zur wählbaren Drehung innerhalb der konvergenten divergenten Venturidüse eines axial strömenden wässrigen Schlammes mit einer Anfangszusammensetzung, die die CO2-reaktiven Ausgangsstoffe und den Eintrittsschlamm umfasst, wobei die Mischer-Rührwerksanordnung bei Auswahl durch einen Leistungsmodulabschnitt umfassend eine richtungswählbare Elektromotoranordnung in Uhrzeiger- und Gegenuhrzeigerrichtung drehbar ist, der verbunden ist, um die Antriebswelle zu betätigen;
mindestens einen Öffnungseintritt in die konvergente divergente Venturidüse, um eine Eintrittsströmung von CO2 in die konvergente divergente Venturidüse bereitzustellen;
mindestens einen Öffnungseitritt in die konvergente divergente Venturidüse, um eine Eintrittsströmung des Eintrittsschlammes in die konvergente divergente Venturidüse bereitzustellen;
- Einführen des axial strömenden wässrigen Schlammes in den konvergenten divergenten Venturidüsenabschnitt durch Eingreifen des Mischer-Rührwerks, um selektiv im Uhrzeiger- und Gegenuhrzeigersinn zu drehen;
- Verarbeiten des Austritts der konvergenten divergenten Venturidüse in einem Lichtabschnitt, umfassend:
einen Tiegel, der aus lichtdurchlässigem Material ausgebildet wird, der mit einem offenen Ende der konvergenten divergenten Venturidüse verbunden ist, wodurch der axial strömende wässrige Schlamm in den Tiegel fließt und mindestens eine Lichtsenderquelle, die in den Tiegel gerichtet ist, um die CO2-reaktiven Ausgangsstoffe des axial strömenden wässrigen Schlammes in Reaktion zu dosieren, um ein Karbonatprodukt herzustellen;
wodurch beim Betrieb des Systems, eintretendes CO₂ reaktiv mit dem axial strömenden wässrigen Schlamm vermischt wird, um kontinuierlich eine Reaktion zu bewirken, die Karbonat-Reaktionsmaterialen als ein Produkt der Ausgangsstoffe zum Resultat hat.

12. Verfahren nach Anspruch 11, wobei die organischen Ausgangsstoffe innerhalb des axial strömenden wässrigen Schlammes in mindestens einem perforierten Zylinder eingeschlossen sind, der in dem Bioreaktor aufgenommen wird, zur Multi-Nukleation von Zellengewebekulturen.

13. Verfahren nach Anspruch 11, wobei mindestens ein Lichtwellenleiter-Strang und mindestens ein Lichtwellenleiterkabel entlang der Innenwände des Bioreaktors montiert sind, um organische Ausgangsstoffe, organische und anorganische Medien sowie anorganische und organische Komponenten des axial strömenden wässrigen Schlammes unter Reaktion in dem Bioreaktor zu dosieren.

14. Verfahren nach Anspruch 11, wobei mindestens ein Lichtwellenleiter-Strang und mindestens ein Lichtwellenleiterkabel entlang der Innenwände des Bioreaktors montiert sind, um organische Ausgangsstoffe, organische und anorganische Medien sowie anorganische und organische Komponenten des axial strömenden wässrigen Schlammes unter Reaktion in dem Bioreaktor zu dosieren.

15. Verfahren nach Anspruch 11, wobei der Bioreaktor ferner mindestens einen Permanentmagneten-Generator umfasst, der um die Axialströmungsturbinen angeordnet ist, um Elektrizität zu erzeugen, um den Energieverbrauch durch den Betrieb des Reaktors auszugleichen.

## Revendications

1. Un système de bioréacteur photolytique destiné à convertir le CO₂ par réaction avec des matières premières réactives au CO₂ pour produire des matériaux de réaction carbonatés comprenant :
- une section de buse venturi comprenant :
une partie centrale de corps de pression, la partie centrale de corps de pression comprenant une buse venturi convergente-divergente s'étendant le long d'un axe horizontal ;
une bande chauffante installée autour d'un diamètre de surface externe d'une section convergente de la buse venturi convergente-divergente ;
un ensemble agitateur mélangeur comprenant une pluralité de turbines à flux axial installées le long de l'axe horizontal sur un arbre d'entraînement pour rotation sélectionnable à l'intérieur de la buse venturi convergente-divergente d'un lisier aqueux à flux axial ayant une composition initiale comprenant les matières premières réactives au CO2 et le lisier d'entrée, l'ensemble agitateur mélangeur étant apte à tourner en direction horaire et antihoraire sur sélection par une partie module d'alimentation comprenant un moteur électrique à direction sélectionnable connecté pour entraîner l'arbre d'entraînement;
au moins une entrée dans la buse venturi convergente-divergente pour fournir un flux d'entrée de CO2 dans la buse venturi convergente-divergente ;
au moins une entrée dans la buse venturi convergente-divergente pour fournir un flux d'entrée du lisier initial dans la buse venturi convergente-divergente ;
- une section lumineuse comprenant :
un creuset formé d'une paroi translucide à la lumière et la section comportant au moins une source émettrice de lumière connectée à une extrémité ouverte de la buse venturi convergente-divergente, de sorte que le lisier aqueux à flux axial s'écoule dans le creuset et que ladite au moins une source émettrice de lumière est dirigée dans le creuset pour doser les matières premières réactives au CO2 du lisier aqueux à flux axial en réaction afin de créer un produit carbonaté ;
- une section module d'alimentation comprenant :
l'arbre d'entraînement s'étendant de la section de buse venturi convergente-divergente à la section module d'alimentation ;
un moteur réversible connecté à l'arbre d'entraînement étant sélectivement actionnable pour une direction de rotation choisie ;
de sorte qu'au fonctionnement du système, le CO₂ entrant s'entremêle de façon réactive avec le lisier initial pour provoquer continuellement une réaction aboutissant à des matériaux de réaction carbonatés en tant que produit des matières premières.

2. Le bioréacteur photolytique selon la revendication 1, dans lequel ladite au moins une source émettrice de lumière comprend une source émettrice de lumière ultraviolette.

3. Le bioréacteur photolytique selon la revendication 1, dans lequel ladite au moins une source émettrice de lumière comprend une source émettrice de lumière visible.

4. Le bioréacteur photolytique selon la revendication 1 comprenant en outre des matières premières organiques dans le lisier aqueux à flux axial qui sont enfermées dans au moins un cylindre perforé logé dans le réacteur, pour la multi-nucléation de cultures de tissus cellulaires.

5. Le bioréacteur photolytique selon la revendication 1 dans lequel au moins une fibre optique et au moins un câble à fibre optique sont montés le long des parois intérieures du bioréacteur dans le but de doser des matières premières organiques, des supports organiques et inorganiques, des composants inorganiques et organiques du lisier aqueux à flux axial en réaction dans le bioréacteur.

6. Le bioréacteur photolytique selon la revendication 1 comprenant également au moins un générateur thermoélectrique destiné à convertir la chaleur résiduelle en électricité.

7. Le bioréacteur photolytique selon la revendication 1 comprenant également au moins un générateur à aimant permanent enfermé autour des turbines à flux axial dans le but de générer de l'électricité pour compenser l'énergie consommée par le fonctionnement du réacteur.

8. Le bioréacteur photolytique selon la revendication 1, dans lequel la section lumineuse comprend en outre une source émettrice de lumière visible pour doser des matières premières sélectionnées du lisier aqueux à flux axial en réaction.

9. Le bioréacteur photolytique selon la revendication 1, dans lequel la source de CO₂ provient au moins d'émissions industrielles.

10. Le bioréacteur photolytique selon la revendication 1, dans lequel la source de CO₂ provient au moins d'émissions industrielles et de CO₂ capté directement dans l'air.

11. Un procédé de production de matériaux réactifs carbonatés comprenant :
- collecter un lisier d'entrée comprenant un lisier initial et du CO₂; et
- traiter le lisier d'entrée collecté dans une section de buse venturi convergente-divergente d'un bioréacteur, le bioréacteur comprenant :
une partie centrale de corps de pression, la partie centrale de corps de pression comprenant une buse venturi convergente-divergente s'étendant le long d'un axe horizontal ;
une bande chauffante installée autour d'un diamètre de surface externe d'une section convergente de la buse venturi convergente-divergente ;
un ensemble agitateur mélangeur comprenant une pluralité de turbines à flux axial installées le long de l'axe horizontal sur un arbre d'entraînement pour rotation sélectionnable à l'intérieur de la buse venturi convergente-divergente d'un lisier aqueux à flux axial ayant une composition initiale comprenant les matières premières réactives au CO2 et le lisier d'entrée, l'ensemble agitateur mélangeur étant apte à tourner en direction horaire et antihoraire sur sélection par une partie module d'alimentation comprenant un moteur électrique à direction sélectionnable connecté pour entraîner l'arbre d'entraînement ;
au moins une entrée dans la buse venturi convergente-divergente pour fournir un flux d'entrée de CO2 dans la buse venturi convergente-divergente ;
au moins une entrée dans la buse venturi convergente-divergente pour fournir un flux d'entrée de la forme de lisier d'entrée dans la buse venturi convergente-divergente ;
- recycler le lisier aqueux à flux axial dans la section de buse venturi convergente-divergente en engageant l'agitateur mélangeur à tourner sélectivement en sens horaire et antihoraire ;
- traiter la sortie de la buse venturi convergente-divergente dans une section lumineuse comprenant :
un creuset de matériau translucide transmissible à la lumière connecté à une extrémité ouverte de la buse venturi convergente-divergente de sorte que le lisier aqueux à flux axial s'écoule dans le creuset et au moins une source émettrice de lumière dirigée dans le creuset pour doser les matières premières réactives au CO2 du lisier aqueux à flux axial en réaction afin de créer un produit carbonaté ;
de sorte qu'au fonctionnement du système, le CO2 entrant s'entremêle de façon réactive avec le lisier aqueux à flux axial pour provoquer continuellement une réaction aboutissant à des matériaux de réaction carbonatés en tant que produit des matières premières.

12. Le procédé selon la revendication 11, dans lequel les matières premières organiques contenues dans le lisier aqueux à flux axial sont enfermées dans au moins un cylindre perforé logé dans le bioréacteur, pour la multi-nucléation de cultures de tissus cellulaires.

13. Le procédé selon la revendication 11, dans lequel au moins une fibre optique et au moins un câble à fibre optique sont montés le long des parois intérieures du bioréacteur dans le but de doser des matières premières organiques, des supports organiques et inorganiques, des composants inorganiques et organiques du lisier aqueux à flux axial en réaction dans le bioréacteur.

14. Le procédé selon la revendication 11, dans lequel le bioréacteur comprend également au moins un générateur thermoélectrique destiné à convertir la chaleur résiduelle en électricité.

15. Le procédé selon la revendication 11, dans lequel le bioréacteur comprend également au moins un générateur à aimant permanent enfermé autour des turbines à flux axial dans le but de générer de l'électricité pour compenser l'énergie consommée par le fonctionnement du réacteur.
